(19) Europäisches Patentamt
European Patent Office
Office européen des brevets

(11) Publication number: **0 292 699 B1**

(12) **EUROPEAN PATENT SPECIFICATION**

(45) Date of publication of patent specification: **23.03.94**

(51) Int. Cl.[5]: **C07C 275/24**, A61K 31/17

(21) Application number: **88106373.9**

(22) Date of filing: **21.04.88**

(54) **Urea based lipoxygenase inhibiting compounds.**

(30) Priority: **24.04.87 US 42491**

(43) Date of publication of application:
**30.11.88 Bulletin 88/48**

(45) Publication of the grant of the patent:
**23.03.94 Bulletin 94/12**

(84) Designated Contracting States:
**BE CH DE ES FR GB GR IT LI NL SE**

(56) References cited:
**EP-A- 0 196 184**
**EP-A- 0 279 263**
**EP-A- 0 279 281**
**US-A- 4 769 387**

**CHEMICAL ABSTRACTS, vol. 98, no. 13, 28th March 1983, page 567, no. 106923s, Columbus, Ohio, US; V.P. TASHCHI et al.: "N-Acylnitrones as intermediates of the reaction of Z-benzaldoxime with isocyanates"**

**CHEMICAL ABSTRACTS, vol. 102, no. 23, 10th June 1985, page 574, no. 203701b, Columbus, Ohio, US; & JP-A-60 08 243**

**CHEMICAL ABSTRACTS, vol. 106, no. 5, 2nd February 1987, page 493, no. 32438x, Columbus, Ohio, US; G. ZINNER et al.: "Observations on an investigation by Ernst Beckmann of reactions of N-benzylhydroxylamines (1987)"**

(73) Proprietor: **ABBOTT LABORATORIES**

**Abbott Park, Illinois 60064(US)**

(72) Inventor: **Summers, James B., Jr.**
**1108 Crestfield Avenue**
**Libertyville Illinois 60048(US)**
Inventor: **Stewart, Andrew O.**
**16235 Arlington Drive**
**Libertyville Illinois 60048(US)**
Inventor: **Brooks, Dee W.**
**1690 Young Drive**
**Libertyville Illinois 60048(US)**

(74) Representative: **Modiano, Guido, Dr.-Ing. et al**
**Baaderstrasse 3**
**D-80469 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

This invention relates to organic compounds which inhibit lipoxygenase enzymes. It also relates to methods of inhibiting lipoxygenase enzymes in human and animal hosts in need of such treatment.

The lipoxygenases are a family of enzymes which catalyze the oxygenation of arachidonic acid The enzyme 5-lipoxygenase converts arachidonic acid to 5-hydroperoxyeicosatetraenoic acid (5-HPETE). This is the first step in the metabolic pathway yielding 5-hydroxyeicosatetraenoic acid (5-HETE) and the important class of mediators, the leukotrienes (LTs).

A variety of biological effects are associated with these products from lipoxygenase metabolism of arachidonic acid and they have been implicated as mediators in various disease states. For example, the LTs $C_4$ and $D_4$ are potent constrictors of human airways *in vitro*, and aerosol administration of these substances to non-asthmatic volunteers induces broncho-constriction. $LTB_4$ and 5-HETE are potent chemotactic factors for inflammatory cells such as polymorphonuclear leukocytes. They also have been found in the synovial fluid of rheumatoid arthritic patients. Leukotrienes have also been implicated as important mediators in allergic rhinitis, psoriasis, adult respiratory distress syndrome, Crohn's disease, endotoxin shock, and ischemia induced myocardial injury among others. The biological activity of the LTs has been reviewed by Lewis and Austen (*J. Clinical Invest.* **73**, 889, 1984) and by J. Sirois (*Adv. Lipid Res.* **21**,78, 1985).

Thus, lipoxygenase enzymes are believed to play an important role in the biosynthesis of mediators of asthma, allergy, arthritis, psoriasis, and inflammation. Blocking these enzymes interrupts the biochemical pathways believed to be involved in these disease states.

Known from EP-A-196 184 are compounds for inhibiting lipoxygenase enzymes among which are disclosed some N-hydroxy-urea derivatives.

EP-A-279 281, which represents state of the art according to Article 54(3) and (4) EPC, discloses some N-hydroxy-urea derivatives useful as inhibitors of 5- and/or 12-lipoxygenase enzymes.

The present invention includes compounds of formula I below, pharmaceutical compositions containing such compounds and the use of such compounds in preparing a medicament for inhibiting 5- and/or 12-lipoxygenase activity in a mammal in need of such treatment

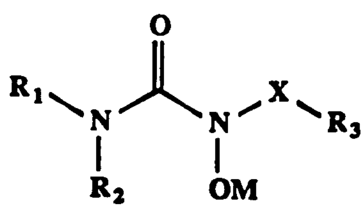

I

$R_1$ and $R_2$ are independently selected from hydrogen, $C_1$ to $C_4$ alkyl or hydroxy, but $R_1$ and $R_2$ are not simultaneously hydroxy.

X is selected from $(C(R_4)_2)_m$, where $m = 1$ to 3 and where $R_4$ is selected independently at each occurrence from hydrogen, or $C_1$ to $C_4$ alkyl.

$R_3$ is

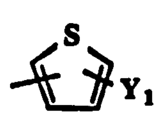

wherein $Y_1$ is selected from hydrogen, $C_1$ to $C_6$ alkyl, $C_2$ to $C_6$ alkenyl, $C_1$ to $C_6$ alkoxy, $C_3$ to $C_8$ cycloalkyl, aryl, aryloxy, aroyl, $C_1$ to $C_6$ arylalkyl, $C_2$ to $C_6$ arylalkenyl, $C_1$ to $C_6$ arylalkoxy, $C_1$ to $C_6$ arylthioalkoxy, or substituted derivatives of aryl, aryloxy, aroyl, $C_1$ to $C_6$ arylalkyl, $C_2$ to $C_6$ arylalkenyl, $C_1$ to $C_6$ arylalkoxy, $C_1$ to $C_6$ arylthioalkoxy, wherein substituents are selected from halo, nitro, cyano, $C_1$ to $C_6$ alkyl, alkoxy, or halosubstituted alkyl.

Finally, M is hydrogen, a pharmaceutically acceptable cation, aroyl, or $C_1$ to $C_6$ alkoyl.

According to this description the groups X and $R_4$ together may form such moieties as $-CH_2-$, $-CHCH_3-$, $-CHC_2H_5-$, $-C(CH_3)_2-$, $-CH(CH_3)CH_2-$, and $-CH_2CH_2CH_2-$.

Disease states which may be treated in humans or lower animal hosts by the compounds described above include, but are not limited to, asthma, rheumatoid arthritis, gout, psoriasis, allergic rhinitis, adult respiratory distress syndrome, Crohn's disease, endotoxin shock, and/or ischemia induced myocardial injury.

An example of a compound which is itself within the scope of the present invention and/or can be used according to the methods of the present invention is N-hydroxy-N-(1-(5-methyl-2-thienyl)ethyl)urea.

The term alkyl is used herein to mean straight and branched chain radicals, including, but not limited to methyl, ethyl, n-propyl, isopropyl, n-butyl, sec-butyl, isobutyl, and tert-butyl.

The term alkenyl is used herein to mean straight and branched chain unsaturated radicals, including, but not limited to ethenyl, 1-propenyl, 2-propenyl, 2-methyl-1-propenyl, 1-butenyl, and 2-butenyl.

The term cycloalkyl is used herein to mean cyclic radicals, preferably of 3 to 8 carbons, including, but not limited to cyclopropyl, cyclobutyl, cyclopentyl, and cyclohexyl.

The term alkoxy is used herein to mean straight and branched chained oxygen ether radicals, including, but not limited to methoxy, ethoxy, isopropoxy, n-butoxy, sec-butoxy, isobutoxy, and tert-butoxy.

The term alkoyl is used herein to mean straight or branched carbonyl radicals, including, but not limited to formyl, acetyl, propionyl, butyryl, isobutyryl, and pivaloyl.

The term aryl is used herein to mean substituted and unsubstituted aromatic radicals, including, but not limited to phenyl, and 1- or 2-naphthyl.

The term aroyl is used herein to mean substituted and unsubstituted aromatic ether radicals, including, but not limited to benzoyl, 1-naphthoyl, and 2-naphthoyl.

The term aryloxy as used herein refers to substituted or unsubstituted aryl ethers including, but not limited to phenoxy, 1-naphthoxy, and 2-naphthoxy.

The term arylalkoxy as used herein refers to substituted or unsubstituted aryl moieties appended to straight chain alkyl ethers including but not limited to phenylmethoxy (i.e., benzyloxy), 1-phenylethoxy, 2-phenylethoxy, 1-naphthylmethyloxy, 2-napthylmethyloxy, 2-, 3- or 4-pyridylmethoxy, and 2-, 3-, 4-, 5-, 6-, 7- or 8-quinolylmethoxy.

The term arylalkyl as used herein refers to substituted or unsubstituted aryl alkyl radicals including but not limited to phenylmethyl (*i.e.*benzyl), 1-phenylethyl, 2-phenylethyl, and 1-naphthylethyl.

The term arylalkenyl as used herein refers to substituted or unsubstituted unsaturated aryl alkyl radicals including, but not limited to phenylethenyl, 3-phenylprop-1-enyl, 3-phenylprop-2-enyl, and 1-naphthylethenyl.

The terms halo and halogen as used herein refer to radicals derived from the elements fluorine, chlorine, bromine, and iodine.

The term halosubstituted alkyl refers to an alkyl radical as described above substituted with one or more halogens, including, but not limited to chloromethyl, trifluoromethyl, and 2,2,2-trichloroethyl.

The term "pharmaceutically acceptable cation" refers to non-toxic cations including but not limited to those based on the alkali and alkaline earth metals, such as sodium, lithium, potassium, and magnesium as well as nontoxic ammonium, quaternary ammonium, and amine cations, including, but not limited to ammonium, tetramethylammonium, tetraethylammonium, methylamine, dimethylamine, trimethylamine, triethylamine, and ethylamine.

This invention also relates to the use of the compounds of formula I for manufacturing a medicament for inhibiting 5-and/or 12-lipoxygenase activity in a human or lower animal host in need of such treatment. The compounds of the present invention may be administered orally, parenterally or topically in dosage unit formulations containing conventional nontoxic pharmaceutically acceptable carriers, adjuvants and vehicles as desired.

The term parenteral as used herein includes subcutaneous, intravenous, intraarterial injection or infusion techniques, without limitation. The term "topically" encompasses administration rectally and by inhalation spray, as well as by the more common routes of the skin and the mucous membranes of the mouth and nose.

Total daily dose of the compounds of this invention administered to a host in single or divided doses may he in amounts, for example, of from about 0.001 to about 100 mg/Kg body weight daily and more usually 0.01 to 10 mg/kg/day. Dosage unit compositions may contain such amounts of such submultiples thereof as may he used to make up the daily dose. It will be understood, however, that the specific dose level for any particular patient will depend upon a variety of factors including the body weight, general health, sex, diet, time and route of administration, rates of absorption and excretion, combination with other drugs and the severity of the particular disease being treated.

This invention also provides for compositions in unit dosage form for the inhibition of 5- or 12-lipoxygenase activity in a human or lower animal host in need of such treatment, comprising a compound of

this invention and one or more nontoxic pharmaceutically acceptable carriers, adjuvants or vehicles. The amount of active ingredient that may he combined with such materials to produce a single dosage form will vary depending upon various factors, as indicated above.

A variety of materials can he used as carriers, adjuvants and vehicles in the composition of this invention, as available in the pharmaceutical arts. Injectable preparations, such as oleaginous solutions, suspensions or emulsions, may he formulated according to known art, using suitable dispersing or wetting agents and suspending agents, as needed. The sterile injectable preparation may employ a nontoxic parenterally acceptable diluent or solvent as, for example, sterile nonpyrogenic water or 1,3-butanediol Among the other acceptable vehicles and solvents that may he employed are 5% dextrose injection, Ringer's injection and isotonic sodium chloride injection (as described in the USP/NF). In addition, sterile, fixed oils are conventionally employed as solvents or suspending media. For this purpose any bland fixed oil may he used, including synthetic mono-, di- or triglycerides. Fatty acids such as oleic acid can also be used in the preparation of injectable compositions.

Suppositories for rectal administration of the compound of this invention can he prepared by mixing the drug with suitable nonirritating excipient such as cocoa butter and polyethylene glycols, which are solid at ordinary temperatures but liquid at body temperature and which therefore melt in the rectum and release the drug.

Solid dosage forms for oral administration include capsules, tablets, pills, troches, lozenges, powders and granules. In such solid dosage forms, the active compound may be admixed with at least one inert diluent such as sucrose, lactose or starch. Such dosage forms may also comprise, as is normal practice, pharmaceutical adjuvant substances, e.g., stearate lubricating agents. In the case of capsules, tablets and pills, the dosage forms may also comprise buffering agents. Solid oral preparations can also be prepared with enteric or other coatings which modulate release of the active ingredients.

Liquid dosage forms for oral administration include pharmaceutically acceptable emulsions, solutions, suspensions, syrups and elixirs containing inert non diluents commonly used in the art, such as water and alcohol. Such compositions may also comprise adjuvants, such as wetting agents, emulsifying suspending, sweetening, flavoring and perfuming agents.

Compounds of this invention can be prepared according to the reaction sequence described in Scheme 1. The sequence illustrates the compound of formula I where $R_1$ and $R_2$ are hydrogen, X is $CHCH_3$, and $R_3$ is phenyl, although such compound (5) is not a compound according to the invention by virtue of the fact that $R_3$ cannot be phenyl.

Compounds of this invention can be prepared in the same manner using the appropriate starting materials.

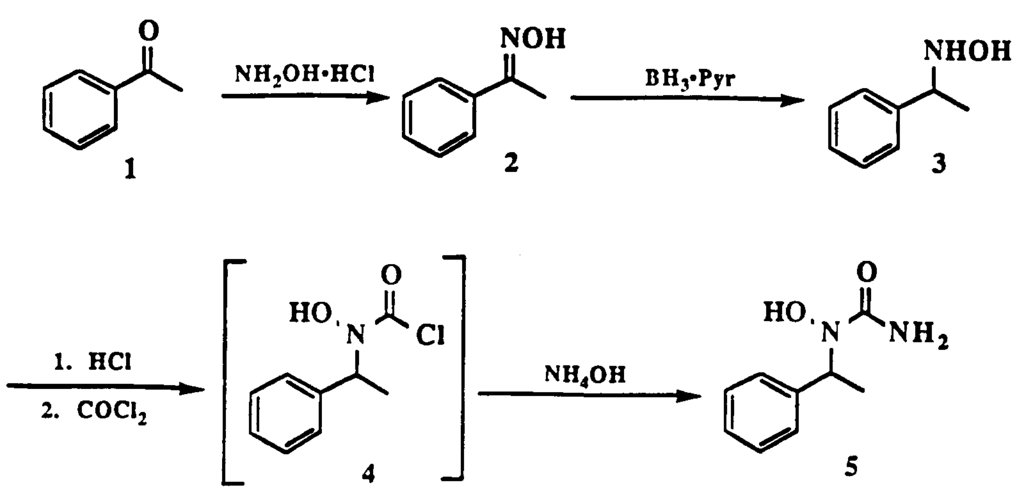


1 2 3 4 5

Scheme 1

Acetophenone (1) is treated with hydroxylamine in ethanol/pyridine to produce the oxime (2). This is reduced to the hydroxylamine (3) with borane pyridine complex and then convened to the hydrochloride salt with gas. Treatment with phosgene yields carbamoyl chloride (4) which is reacted without isolation to yield the urea (5). Other reagents may also be used to carry out the same transformations. For example, 2 may be converted to 3 using borane dimethylamine or other borane amine complexes or with sodium

cyanoborohydride.

Compounds of formula I can also be prepared according to Scheme 2, below. The sequence illustrates the case where $R_1$ and $R_2$ are hydrogen and where $R_3$ is phenyl and X is $CH(CH_3)$, although such compound (5) is not a compound according to the invention by virtue of the fact that $R_3$ cannot be phenyl.

Compounds of this invention can also be prepared in this manner

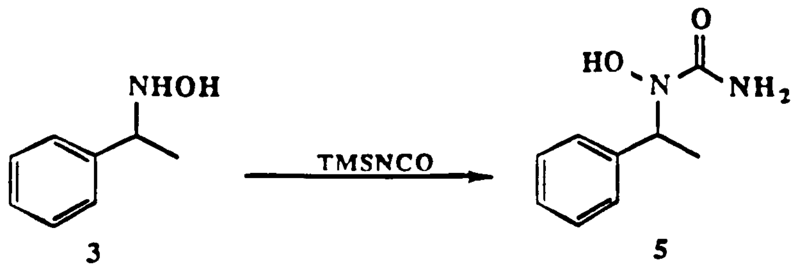


3 5

Scheme 2

Hydroxylamine (3) is treated with trimethylsilylisocyanate, followed by ammonium chloride workup to give the urea (5).

In addition to the methods described above the compounds of this invention may also be prepared according to the method of scheme 3 with the appropriate substitution of the phenyl group by $R_3$ of formula I.

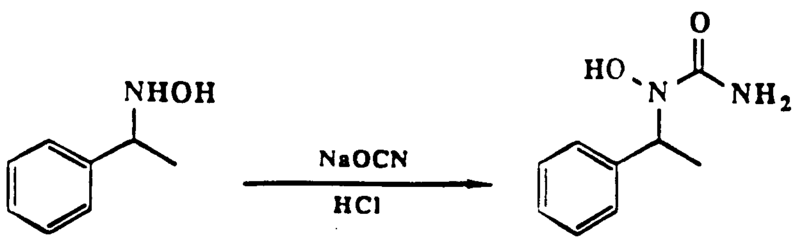


3 5

Scheme 3

The hydroxylamine (3) is dissolved in dilute hydrochloric acid and aqueous sodium cyanate is added. The resulting urea (5) precipitates from the reaction and is collected.

In addition to the method described in scheme 1, above hydroxylamine (3) can also be prepared according to the method of scheme 4, below. The sequence illustrates the case where $R_1$ and $R_2$ are hydrogen and where $R_3$ is phenyl and X is $CH(CH_3)$, although such intermediate (3) does not lead to a compound (5) of the invention by virtue of the fact that $R_3$ cannot be phenyl.

Compounds of this invention can also be prepared in this manner with the appropriate substitution of the phenyl group by $R_3$ of formula I.

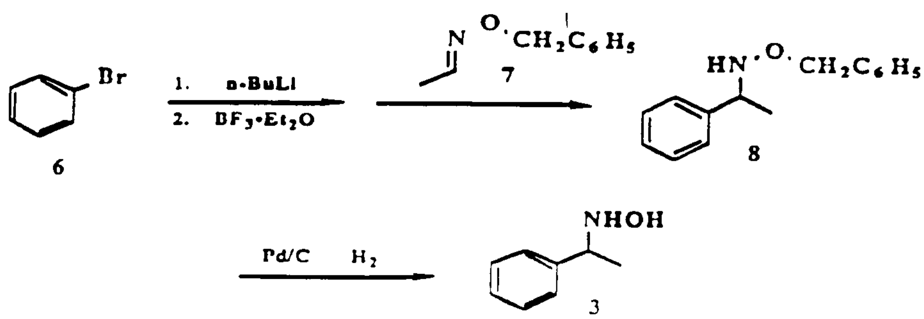


6 7 8 3

Scheme 4

Bromobenzene (6) is converted to phenyl lithium by treatment with n-butyl lithium at -78°C. Boron trifluoride etherate is then added and this is reacted with acetaldehyde O-benzyl oxime (7) to yield (8). The O-benzyl protecting group is removed by catalytic hydrogenation. Other reagents may be substituted for those described above. For example, phenyl lithium maybe prepared by treatment with t-butyl lithium, sec-butyl lithium, or lithium metal instead of n-butyl lithium. Other protecting groups may be used for acetaldehyde oxime. For example, benzyloxymethoxy, methyloxymethoxy, methoxybenzyl may be used instead of benzyl.

The following examples further illustrate the synthesis and use of compounds to this invention. The appropriate designations of $R_1$, $R_2$, $R_3$ and X as defined by formula I are designated for each example. Example 1 is given for purposes of illustration only and does not involve a compound of the invention.

**Example 1**

**N-hydroxy-N-(1-(4-phenylmethoxyphenyl)ethyl) urea**

a. 4-Phenylmethoxyacetophenone. 4-Hydroxyacetophenone (5.0 g, 36.7 mmole) was dissolved in dimethylsulfoxide (50 mL) and potassium t-butoxide (4.73 g, 42.2 mmole) was added. Twenty minutes later benzyl bromide (7.85g, 45.8 mmole) was added. After an additional hour, the reaction mixture was poured into water and extracted with ether. The ether layer was dried with magnesium sulfate and evaporated to give an off white solid which was carried on without further purification.

b. 4-Phenylmethoxyacetophenone oxime. Using the method described in scheme 1, the materian prepared as in part a, above and hydroxylamine hydrochloride (4.8 g, 0.70 mole) were dissolved in a mixture of ethanol (25 mL) and pyridine (25mL) and heated at 50° for 2 hours. Most of the solvent was removed *in vacuo* and the residue dissolved in ether. After washing with 2N HCl (50 mL), the solution was dried over $MgSO_4$ and evaporated. A white crystalline solid was obtained and was carried on without further purification.

An alternative work-up may also be used. The reaction mixture is diluted with water (300 mL) and the product precipitates. It is filtered off and dried*in vacuo*.

c. 1-(4-Phenylmethoxyphenyl) ethyl hydroxyl amine. 4-Phenylmethoxy acetophenone oxime (3.7 g 15.3 mmole), prepared as described in part b above, was dissolved in ethanol (30 mL) and cooled to 0°C. Borane-pyridine complex (4.6 mL, 46 mmole) was added *via* syringe under nitrogen followed ten minutes later by 6N HCl (15 mL). Within thirty minutes the reaction was complete and was brought to pH 9 with the addition of solid sodium carbonate or 2N NaOH. The mixture was extracted into ether and dried over $MgSO_4$. After evaporation a white solid resulted which was carried on without further purification.

d. N-hydroxy-N-(1-(4-phenylmethoxyphenyl)ethyl) urea. *Method a.* Using the method of scheme 2, the material prepared as in part a above (2g, 8.2 mmole) was refluxed for thirty minutes with trimethylsilyl isocyanate (0.95 g, 8.2 mmole) in 30 mL dioxane. The reaction mixture was then washed with saturated $NH_4Cl$ solution, dried with $MgSO_4$, and evaporated. The residue was washed with ether to give 1.33g of a white solid.

*Method b*. Using the method of scheme 1, the material prepared as in part a above (4g, 16.4 mmole) was dissolved in toluene (100 mL) and HCl gas was bubbled through the mixture at a moderate rate for about four minutes. The solution was then heated to reflux and phosgene was bubbled through for another four minutes. After an additional one hour reflux, the mixture was allowed to cool to room temperature and then added to excess cold ammonium hydroxide solution. The precipitate was collected and recrystallized from aqueous ethanol ($R_1$,$R_2$ = H, $R_3$ = 4-$C_6H_5CH_2OC_6H_4$, X = CH($CH_3$)).

Melting Point: 132-134°C.

NMR (300 MHz, DMSO-$d_6$): 1.37 (d, 3H); 5.08 (s, 2H); 5.24 (q, 1H); 6.25 (s, 2H); 6.92 (d, 2H); 7.25 (d, 2H); 7.30-7.50 (m, 5H); 8.90 (s, 1H).

Mass spectrum (EI): 286 $M^+$, 269, 255, 243, 226, 211, 91.

**Example 2**

**N-hydroxy-N-(1-(5-methyl-2-thienyl)ethyl) urea**

The desired material was prepared according to the method of example 1, except using 5-methyl-2-acetyl thiophenene instead of 4-phenylmethoxyacetophenone ($R_1$, $R_2$ = H, $R_3$ = 5-$CH_3$-2-thienyl, X = CH-($CH_3$)).

NMR (300 MHz, DMSO-$d_6$): 1.38 (d, 3H); 2.38 (s, 3H); 5.39 (q, 1H); 6.36 (brs, 2H); 6.59 (m, 1H); 6.70 (m, 1H); 9.08 (s, 1H).

Mass spectrum (CI-$NH_3$): 218 $(M+NH_4)^+$,201 $(M+1)^+$,125.

**Lipoxygenase $IC_{50}$ Determination**

Assays to determine 5-lipoxygenase activity were performed in 200ml incubations containing the 20,000xg supernatant from 1.5 million homogenized RBL-1 cells and various concentrations of the test compound. Reactions were initiated by addition of radiolabeled arachidonic acid and terminated by acidification and ether extraction. Reaction products were separated from nonconverted substrate by thin layer chromatography and measured by liquid scintillation spectroscopy. All incubations are performed in triplicate. Inhibition of 5-lipoxygenase activity was calculated as the ratio of the amount of product formed in the presence and absence of inhibitor. $IC_{50}$ values and 95% confidence limits were computed from linear regression analysis of percentage inhibition versus log concentration plots. Results for the compound of Example 2 are indicated in Table 1.

**Table 1. *In Vitro* Inhibitory Potency of a Compound of this Invention Against 5-Lipoxygenase from RBL-1 20,000xg Supernatant**

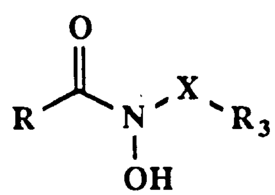

| Example | R | X | $R_3$ | $IC_{50}$ (μM) |
|---|---|---|---|---|
| 2 | $NH_2$ | $CHCH_3$ | 5-methyl-2-thienyl | 2.8 |

**Claims**

**Claims for the following Contracting States : BE, CH, DE, FR, GB, IT, LI, NL, SE**

**1.** A compound of the formula:

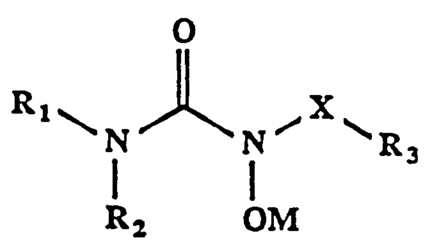

I

wherein $R_1$ and $R_2$ are independently selected from hydrogen, $C_1$ to $C_4$ alkyl, or hydroxy, but $R_1$ and $R_2$ are not simultaneously hydroxy;

wherein X is selected from $(C(R_4)_2)_m$, where m = 1 to 3;

wherein $R_4$ is selected independently at each occurrence from hydrogen, or $C_1$ to $C_4$ alkyl,

wherein $R_3$ is

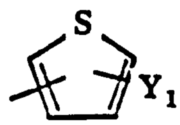

wherein $Y_1$ is selected from hydrogen, $C_1$ to $C_6$ alkyl, $C_2$ to $C_6$ alkenyl, $C_1$ to $C_6$ alkoxy, $C_3$ to $C_8$ cycloalkyl, aryl, aryloxy, aroyl, $C_1$ to $C_6$ arylalkyl, $C_2$ to $C_6$ arylalkenyl, $C_1$ to $C_6$ arylalkoxy, $C_1$ to $C_6$ arylthioalkoxy, or substituted derivatives of aryl, aryloxy, aroyl, $C_1$ to $C_6$ arylalkyl, $C_2$ to $C_6$ arylalkenyl, $C_1$ to $C_6$ arylalkoxy, $C_1$ to $C_6$ arylthioalkoxy, wherein substituents are selected from halo, nitro, cyano, $C_1$ to $C_6$ alkyl, alkoxy, or halosubstituted alkyl;

and finally wherein M is hydrogen, a pharmaceutically acceptable cation, aroyl, or $C_1$ to $C_6$ alkoyl.

**2.** The compound according to claim 1 wherein $R_1$ and $R_2$ are both hydrogen.

**3.** The compound according to claim 1 wherein $R_1$ is hydrogen and $R_2$ is hydroxy.

**4.** The compound according to claim 1 wherein X is $CH(CH_3)$.

**5.** The compound according to claim 1 wherein X is $CH_2$.

**6.** The compound according to claim 1 wherein $Y_1$ is phenylmethoxy

**7.** The compound according to claim 1 wherein $Y_1$ is butoxy.

**8.** A composition for the inhibition of 5- or 12-lipoxygenase activity in a human or lower animal host in need of such treatment, comprising a compound according to Claim 1 and one or more nontoxic pharmaceutically acceptable carriers, adjuvants or vehicles.

**9.** A composition for treating asthma, allergic rhinitis, rheumatoid arthritis, gout, adult respiratory distress syndrome, Crohn's disease, psoriasis, endotoxin shock, and/or ischemia-induced myocardial injury in a human and lower animal in need of such treatment, comprising a compound according to Claim 1 and one or more nontoxic pharmaceutically acceptable carriers, adjuvants or vehicles.

**10.** Use of a compound according to Claim 1 for preparing a medicament for inhibiting 5- or 12-lipoxygenase activity in a human or lower animal host in need of such treatment.

**11.** Use of a compound according to Claim 1 for preparing a medicament for treating asthma, allergic rhinitis, rheumatoid arthritis, gout, adult respiratory distress syndrome, Crohn's disease, psoriasis, endotoxin shock, and/or ischemia-induced myocardial injury in a human or lower animal in need of such treatment.

**Claims for the following Contracting States : ES, GR**

**1.** A process for preparing a compound of the formula:

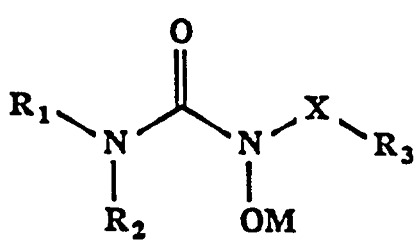

I

wherein $R_1$ and $R_2$ are independently selected from hydrogen, $C_1$ to $C_4$ alkyl, or hydroxy, but $R_1$ and $R_2$ are not simultaneously hydroxy;

wherein X is selected from $(C(R_4)_2)_m$, where m = 1 to 3;

wherein $R_4$ is selected independently at each occurence hydrogen, or $C_1$ to $C_4$ alkyl,

wherein $R_3$ is

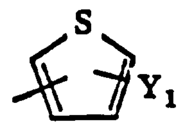

wherein $Y_1$ is selected from hydrogen, $C_1$ to $C_6$ alkyl, $C_2$ to $C_6$ alkenyl, $C_1$ to $C_6$ alkoxy, $C_3$ to $C_8$ cycloalkyl, aryl, aryloxy, aroyl, $C_1$ to $C_6$ arylalkyl, $C_2$ to $C_6$ arylalkenyl, $C_1$ to $C_6$ arylalkoxy, $C_1$ to $C_6$ arylthioalkoxy, or substituted derivatives of aryl, aryloxy, aroyl, $C_1$ to $C_6$ arylalkyl, $C_2$ to $C_6$ arylalkenyl, $C_1$ to $C_6$ arylalkoxy, $C_1$ to $C_6$ arylthioalkoxy, wherein substituents are selected from halo, nitro, cyano, $C_1$ to $C_6$ alkyl, alkoxy, or halosubstituted alkyl;

and finally, wherein M is hydrogen, a pharmaceutically acceptable cation, aroyl, or $C_1$ to $C_6$ alkoyl;

said process comprising reacting a carbamoyl chloride of the formula:

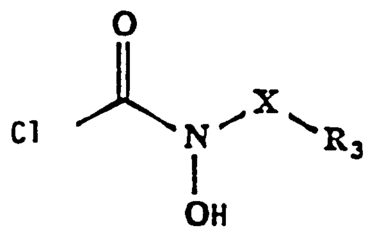

wherein X and $R_3$ are as defined above, with an amine of the formula $HNR_1R_2$ wherein $R_1$ and $R_2$ are as defined above to provide a compound of the formula:

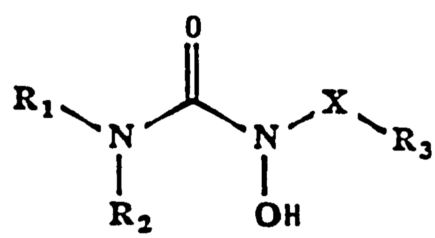

which, when M is not hydrogen, is converted to a salt or ester of the formula:

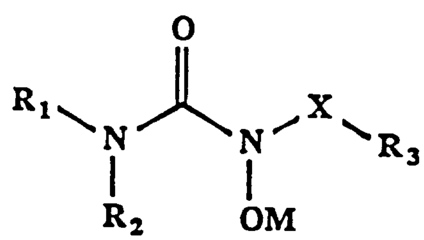

wherein M is a pharmaceutically acceptable cation, aroyl or $C_1$ to $C_6$ alkoyl.

2. The process of Claim 1 wherein the carbamoyl chloride of formula:

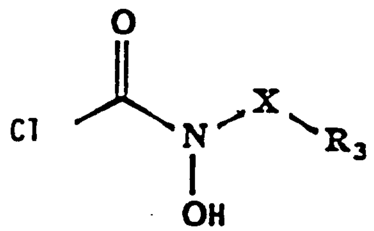

wherein X and $R_3$ are as defined above is prepared by a process comprising reacting a hydroxylamine of the formula:

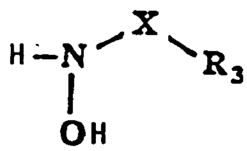

wherein X and $R_3$ are as defined above, with a carbamoyl chloride forming agent to provide a compound of the formula:

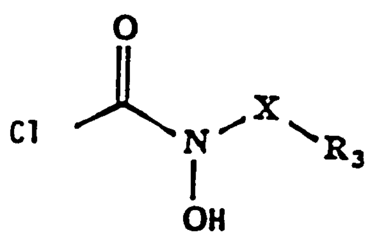

wherein X, $R_1$, $R_2$ and $R_3$ are as defined above.

3. The process of Claim 2 wherein the hydroxylamine of formula:

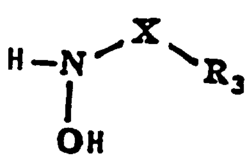

wherein X and $R_3$ are as defined above is prepared by a process comprising converting a ketone or aldehyde of the formula $O=X\text{-}R_3$ wherein X and $R_3$ are as defined above to an oxime of the formula $HO\text{-}N=X\text{-}R_3$ wherein X and $R_3$ are as defined above, followed by reduction of the oxime to the hydroxylamine of the formula

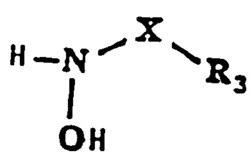

wherein X and $R_3$ are as defined above.

4. The process of Claim 2 wherein the carbamoyl chloride forming agent is phosgene.

5. The process of Claim 3 wherein the ketone or aldehyde is converted to the oxime by treatment with hydroxylamine hydrochloride.

6. The process of Claim 3 wherein the oxime is reduced to the hydroxylamine with borane-pyridine complex.

7. The process of Claim 1 wherein $R_1$ and $R_2$ are both hydrogen.

8. The process of Claim 1 wherein $R_1$ is hydrogen and $R_2$ is hydroxy.

9. A process for preparing a compound of the formula:

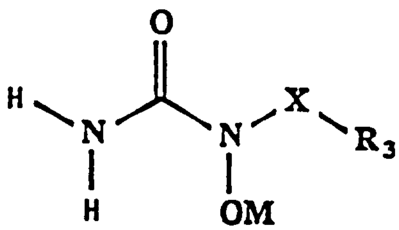

wherein X is selected from $(C(R_4)_2)_m$, where m = 1 to 3;
wherein $R_4$ is selected independently at each occurence from hydrogen, or $C_1$ to $C_4$ alkyl,
wherein $R_3$ is

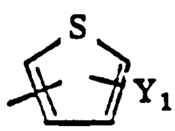

wherein $Y_1$ is selected from hydrogen, $C_1$ to $C_6$ alkyl, $C_2$ to $C_6$ alkenyl, $C_1$ to $C_6$ alkoxy, $C_3$ to $C_8$ cycloalkyl, aryl, aryloxy, aroyl, $C_1$ to $C_6$ arylalkyl, $C_2$ to $C_6$ arylalkenyl $C_1$ to $C_6$ arylalkoxy, $C_1$ to $C_6$ arylthioalkoxy, or substituted derivatives of aryl, aryloxy, aroyl, $C_1$ to $C_6$ arylalkyl, $C_2$ to $C_6$ arylalkenyl, $C_1$ to $C_6$ arylalkoxy, $C_1$ to $C_6$ arylthioalkoxy, wherein substituents are selected from halo, nitro, cyano, $C_1$ to $C_6$ alkyl, alkoxy, or halosubstituted alkyl;
and finally wherein M is hydrogen, a pharmaceutically acceptable cation, aroyl, or $C_1$ to $C_6$ alkoyl;
said process comprising reacting a carbamoyl chloride of the formula:

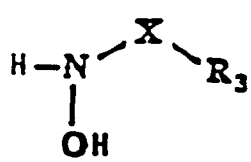

wherein X and $R_3$ are as defined above to a hydroxy urea of the formula:

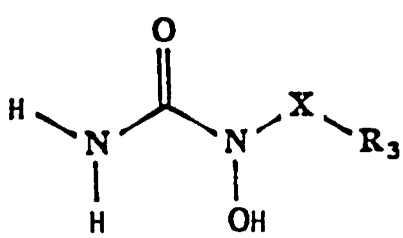

wherein X and $R_3$ are as defined above which, when M is not hydrogen, is converted to a salt or ester of the formula:

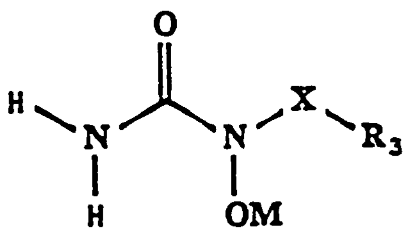

wherein M is a pharmaceutically acceptable cation, aroyl or $C_1$ to $C_6$ alkoyl.

**10.** The process of Claim 9 wherein the hydroxylamine is converted to the hydroxy urea by treatment with sodium cyanate.

**11.** The process of Claim 9 wherein the hydroxylamine is converted to the hydroxy urea by treatment with trimethylsilylisocyanate.

**12.** The process of Claim 9 wherein the hydroxylamine of formula:

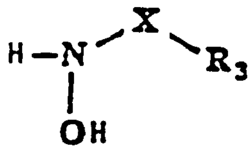

wherein X and $R_3$ are as defined above is prepared by a process comprising converting a ketone or aldehyde of the formula $O=X\text{-}R_3$ wherein X and $R_3$ are as defined above to an oxime of the formula $HO\text{-}N=X\text{-}R_3$ wherein X and $R_3$ are as defined above, followed by reduction of the oxime to the hydroxylamine of the formula

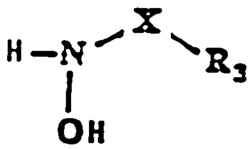

wherein X and $R_3$ are as defined above.

**13.** The process of Claim 12 wherein the ketone or aldehyde is converted to the oxime by treatment with hydroxylamine hydrochloride.

**14.** The process of Claim 12 wherein the oxime is reduced to the hydroxylamine with borane-pyridine complex.

**15.** The process of Claim 1 or 9 wherein X is $CH(CH_3)$.

**16.** The process of Claim 1 or 9 wherein X is $CH_2$.

**17.** The process of Claim 1 or 9 wherein $Y_1$ is phenylmethoxy.

**18.** The process of Claim 1 or 9 wherein $Y_1$ is butoxy.

**19.** Use of a compound of the formula:

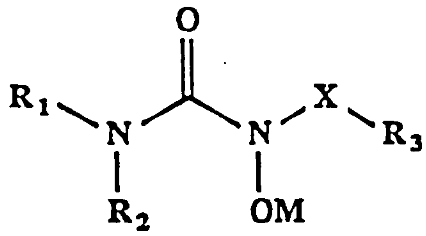

I

wherein $R_1$ and $R_2$ are independently selected from hydrogen, $C_1$ to $C_4$ alkyl, or hydroxy, but $R_1$ and $R_2$ are not simultaneously hydroxy;

wherein X is selected from $(C(R_4)_2)_m$, where m = 1 to 3;

wherein $R_4$ is selected independently at each occurence from hydrogen, or $C_1$ to $C_4$ alkyl,

wherein $R_3$ is

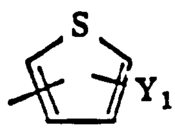

wherein $Y_1$ is selected from hydrogen, $C_1$ to $C_6$ alkyl, $C_2$ to $C_6$ alkenyl, $C_1$ to $C_6$ alkoxy, $C_3$ to $C_8$ cycloalkyl, aryl, aryloxy, aroyl, $C_1$ to $C_6$ arylalkyl, $C_2$ to $C_6$ arylalkenyl, $C_1$ to $C_6$ arylalkoxy, $C_1$ to $C_6$ arylthioalkoxy, or substituted derivatives of aryl, aryloxy, aroyl, $C_1$ to $C_6$ arylalkyl, $C_2$ to $C_6$ arylalkenyl, $C_1$ to $C_6$ arylalkoxy, $C_1$ to $C_6$ arylthioalkoxy, wherein substituents are selected from halo, nitro, cyano, $C_1$ to $C_6$ alkyl, alkoxy, or halosubstituted alkyl;

and finally wherein M is hydrogen, a pharmaceutically, acceptable cation, aroyl, or $C_1$ to $C_6$ alkoyl,

for preparing a medicament for inhibiting 5- or 12-lipoxygenase in a human or lower animal in need of such treatment.

**20.** Use of a compound of the formula:

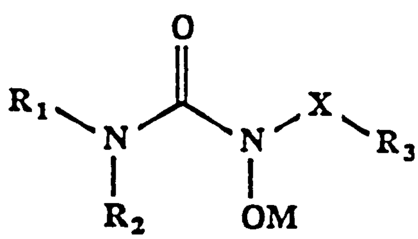

I

wherein $R_1$ and $R_2$ are independently selected from hydrogen, $C_1$ to $C_4$ alkyl, or hydroxy, but $R_1$ and $R_2$ are not simultaneously hydroxy;

wherein X is selected from $(C(R_4)_2)_m$, where m = 1 to 3;

wherein $R_4$ is selected independently at each occurence from hydrogen, or $C_1$ to $C_4$ alkyl,

wherein $R_3$ is

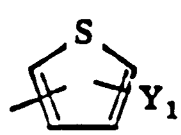

wherein $Y_1$ is selected from hydrogen, $C_1$ to $C_6$ alkyl, $C_2$ to $C_6$ alkenyl, $C_1$ to $C_6$ alkoxy, $C_3$ to $C_8$

cycloalkyl, aryl, aryloxy, aroyl, $C_1$ to $C_6$ arylalkyl, $C_2$ to $C_6$ arylalkenyl, $C_1$ to $C_6$ arylalkoxy, $C_1$ to $C_6$ arylthioalkoxy, or substituted derivatives of aryl, aryloxy, aroyl, $C_1$ to $C_6$ arylalkyl $C_2$ to $C_6$ arylalkenyl, $C_1$ to $C_6$ arylalkoxy, $C_1$ to $C_6$ arylthioalkoxy, wherein substituents are selected from halo, nitro, cyano, $C_1$ to $C_6$ alkyl, alkoxy, or halosubstituted alkyl;

and finally wherein M is hydrogen, a pharmaceutically acceptable cation, aroyl, or $C_1$ to $C_6$ alkoyl;

for preparing a medicament for treating asthma, allergic rhinitis, rheumatoid arthritis, gout, adult respiratory distress syndrome, Crohn's disease, psoriasis, endotoxin shock, and/or ischemia-induced myocardial injury in a human or lower animal in need of such treatment.

## Patentansprüche

## Patentansprüche für folgende Vertragsstaaten : BE, CH, DE, FR, GB, IT, LI, NL, SE

1. Eine Verbindung der Formel:

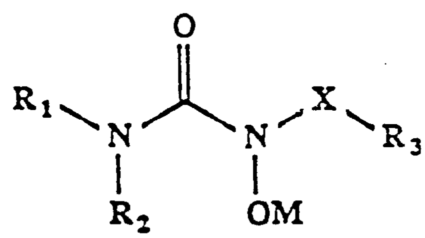

I

wobei $R_1$ und $R_2$ unabhängig voneinander aus Wasserstoff, $C_1$- bis $C_4$-Alkyl oder Hydroxyl gewählt sein können, aber $R_1$ und $R_2$ sind nicht beide gleichzeitig Hydroxyl;
wobei X aus $(C(R_4)_2)_m$ auswählbar ist, mit $m = 1$ bis 3, und mit $R_4$, welcher bei jedem Auftreten unabhängig aus Wasserstoff oder $C_1$- bis $C_4$-Alkyl wählbar ist;
wobei $R_3$

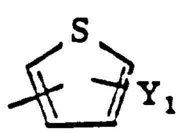

ist, mit $Y_1$ wählbar aus Wasserstoff, $C_1$- bis $C_6$-Alkyl, $C_2$- bis $C_6$-Alkenyl, $C_1$- bis $C_6$-Alkoxy, $C_3$- bis $C_8$-Cycloalkyl, Aryl, Aryloxy, Aroyl, $C_1$- bis $C_6$-Arylalkyl, $C_2$- bis $C_6$-Arylalkenyl, $C_1$- bis $C_6$-Arylalkoxy, $C_1$- bis $C_6$-Arylthioalkoxy, oder substituierte Derivate von Aryl, Aryloxy, Aroyl, $C_1$- bis $C_6$-Arylalkyl, $C_2$- bis $C_6$-Arylalkenyl, $C_1$- bis $C_6$-Arylalkoxy, $C_1$- bis $C_6$-Arylthioalkoxy, wobei die Substituenten aus Halogen, Nitro, Cyano, $C_1$- bis $C_6$-Alkyl, Alkoxy oder halogensubstituiertem Alkyl zu wählen sind;
und wobei schließlich M Wasserstoff, ein pharmazeutisch verträgliches Kation, Aroyl oder $C_1$- bis $C_6$-Alkoyl ist.

2. Die Verbindung gemäß Anspruch 1, wobei $R_1$ und $R_2$ beide Wasserstoff sind.

3. Die Verbindung gemäß Anspruch 1, wobei $R_1$ Wasserstoff und $R_2$ Hydroxy ist.

4. Die Verbindung gemäß Anspruch 1, wobei X $CH(CH_3)$ist.

5. Die Verbindung gemäß Anspruch 1, wobei X $CH_2$ ist.

6. Die Verbindung gemäß Anspruch 1, wobei $Y_1$ Phenylmethoxy ist.

7. Die Verbindung gemäß Anspruch 1, wobei $Y_1$ Butoxy ist.

8. Eine Zusammensetzung zur Inhibition der 5- oder 12-Lipoxygenase-Aktivität in einem menschlichen oder niederem tierischen Wirtsorganismus, der eine solche Behandlung benötigt, die eine Verbindung

gemäß Anspruch 1 und einen oder mehrere nichttoxische, pharmazeutisch verträgliche Trägerstoffe, Hilfsstoffe oder Transportsubstanzen umfaßt.

**9.** Eine Zusammensetzung zur Behandlung von Asthma, allergischer Rhinitis, rheumatoider Arthritis, Gicht, dem Atemdisstressyndrom Erwachsener, dem Crohn'schen Leiden, Psoriase, endotoxinem Schock und/oder Ischämie-induziertem myocardischen Beschwerden bei einem Menschen oder einem niederem Tier im Bedarfsfall einer solchen Behandlung, die eine Verbindung gemäß Anspruch 1 und einen oder mehrere nichttoxische, pharmazeutisch verträgliche Trägerstoffe, Hilfsstoffe oder Transportsubstanzen umfaßt.

**10.** Die Verwendung einer Verbindung gemäß dem Anspruch 1 zur Bereitung eines Medikamentes zur Inhibition der 5- oder 12- Lipoxygenase-Aktivität in einem menschlichen oder niederem tierischen Wirtsorganismus im Bedarfsfall einer solchen Behandlung.

**11.** Die Verwendung einer Verbindung gemäß dem Anspruch 1 zur Bereitung eines Medikaments zur Behandlung von Asthma, allergischer Rhinitis, rheumatoider Arthritis, Gicht, dem Atemdisstressyndrom Erwachsener, dem Crohn'schen Leiden, Psoriase, endotoxinem Schock und/oder Ischämie-induziertem myocardischen Beschwerden bei einem Menschen oder einem niederem Tier im Bedarfsfall einer solchen Behandlung.

**Patentansprüche für folgende Vertragsstaaten : ES, GR**

**1.** Ein Verfahren zur Darstellung einer Verbindung der Formel:

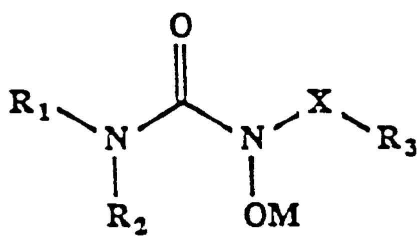

I

wobei $R_1$ und $R_2$ unabhängig voneinander aus Wasserstoff, $C_1$- bis $C_4$-Alkyl oder Hydroxyl gewählt sein können, aber $R_1$ und $R_2$ sind nicht beide gleichzeitig Hydroxyl;
wobei X aus $(C(R_4)_2)_m$ auswählbar ist, mit $m = 1$ bis 3, und mit $R_4$, welcher bei jedem Auftreten unabhängig aus Wasserstoff oder $C_1$- bis $C_4$-Alkyl wählbar ist;
wobei $R_3$

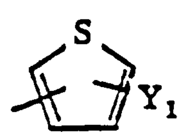

ist, mit $Y_1$ wählbar aus Wasserstoff, $C_1$- bis $C_6$-Alkyl, $C_2$- bis $C_6$-Alkenyl, $C_1$- bis $C_6$-Alkoxy, $C_3$- bis $C_8$-Cycloalkyl, Aryl, Aryloxy, Aroyl, $C_1$- bis $C_6$-Arylalkyl, $C_2$- bis $C_6$-Arylalkenyl, $C_1$- bis $C_6$-Arylalkoxy, $C_1$- bis $C_6$-Arylthioalkoxy, oder substituierte Derivate von Aryl, Aryloxy, Aroyl, $C_1$- bis $C_6$-Arylalkyl, $C_2$- bis $C_6$-Arylalkenyl, $C_1$- bis $C_6$-Arylalkoxy, $C_1$- $C_6$-Arylthioalkoxy wobei die Substituenten aus Halogen, Nitro, Cyano, $C_1$- bis $C_6$-Alkyl, Alkoxy oder halogensubstituiertem Alkyl zu wählen sind;
und wobei schließlich M Wasserstoff, ein pharmazeutisch verträgliches Kation, Aroyl oder $C_1$- bis $C_6$-Alkoyl ist,

wobei das Verfahren die Umsetzung eines Carbamoylchlorides der Formel:

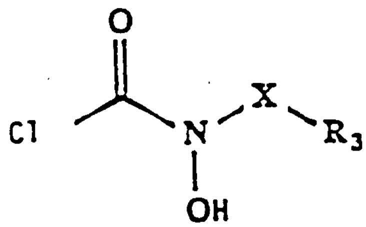

, wobei X und $R_3$ obiger Definition nachkommen, mit einem Amin der Formel $HNR_1R_2$, wobei $R_1$ und $R_2$ obiger Defintion entsprechen, zur Bereitstellung einer Verbindung der Formel:

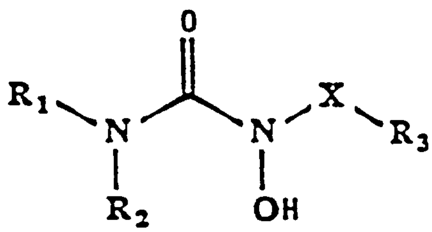

umfaßt, welche, sofern M nicht Wasserstoff ist, in ein Salz oder einen Ester der Formel:

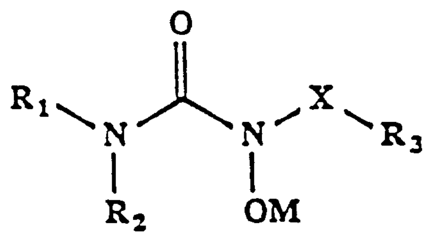

, wobei M ein pharmazeutisch verträgliches Kation, Aroyl oder $C_1$- bis $C_6$-Alkoyl ist, überführt wird.

2. Das Verfahren gemäß Anspruch 1, wobei das Carbamoylchlorid der Formel:

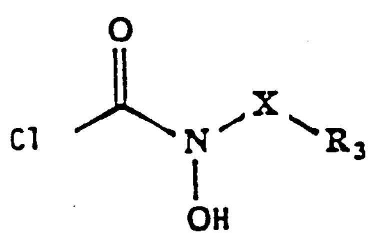

, wobei X und $R_3$ obiger Definition entsprechen, durch ein Verfahren dargestellt wird, welches die Umsetzung eines Hydroxylamins der Formel:

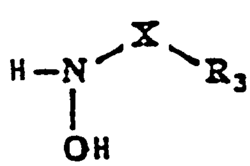

, wobei X und $R_3$ obiger Defintion entsprechen, mit einem Carbamoylchlorid umfaßt, welches ein Agens zur Bereitstellung einer Verbindung der Formel:

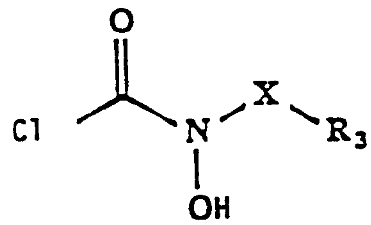

, darstellt, wobei X, $R_1$, $R_2$, und $R_3$ obiger Definition entsprechen.

**3.** Das Verfahren gemäß Anspruch 2, wobei das Hydroxylamin der Formel:

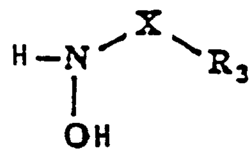

, wobei X und $R_3$ obiger Defintion entsprechen, mittels eines Verfahrens dargestellt wird, welches die Umwandlung eines Ketons oder Aldehyds der Formel $O=X-R_3$, wobei X und $R_3$ obiger Definition entsprechen, gefolgt von der Reduktion des Oxims zum Hydroxylamin der Formel:

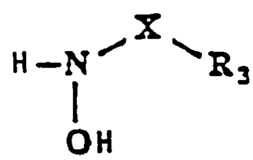

wobei X und $R_3$ obiger Definition entsprechen, umfaßt.

**4.** Das Verfahren gemäß Anspruch 2, wobei das das Carbamoylchlorid bildende Agens Phosgen ist.

**5.** Das Verfahren gemäß Anspruch 3, wobei das Keton oder der Aldehyd durch Behandlung mit Hydroxylaminhydrochlorid in das Oxim überführt wird.

**6.** Das Verfahren gemäß Anspruch 3, wobei das Oxim mittels eines Boran-Pyridin-Komplexes zum Hydroxylamin reduziert wird.

**7.** Das Verfahren gemäß Anspruch 1, wobei $R_1$ und $R_2$ beide Wasserstoff sind.

**8.** Das Verfahren gemäß Anspruch 1, wobei $R_1$ Wasserstoff und $R_2$ Hydroxyl ist.

**9.** Ein Verfahren zur Darstellung einer Verbindung der Formel:

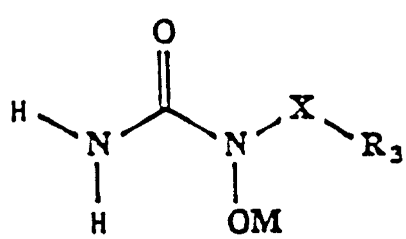

, wobei X aus $(C(R_4)_2)_m$ gewählt ist, wobei m = 1 bis 3 ist; wobei $R_4$ bei jedem Vorkommen unabhängig aus Wasserstoff oder $C_1$- bis $C_4$-Alkyl gewählt ist,

wobei $R_3$

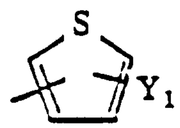

ist, mit $Y_1$ wählbar aus Wasserstoff, $C_1$- bis $C_6$-Alkyl, $C_2$- bis $C_6$-Alkenyl, $C_1$- bis $C_6$-Alkoxy, $C_3$- bis $C_8$-Cycloalkyl, Aryl, Aryloxy, Aroyl, $C_1$- bis $C_6$-Arylalkyl, $C_2$- bis $C_6$-Arylalkenyl, $C_1$- bis $C_6$-Arylalkoxy, $C_1$- bis $C_6$-Arylthioalkoxy, oder substituierte Derivate von Aryl, Aryloxy, Aroyl, $C_1$- bis $C_6$-Arylalkyl, $C_2$- bis $C_6$-Arylalkenyl, $C_1$ bis $C_6$-Arylalkoxy, $C_1$- bis $C_6$-Arylthioalkoxy, wobei die Substituenten aus Halogen, Nitro, Cyano, $C_1$- bis $C_6$-Alkyl, Alkoxy oder halogensubstituiertem Alkyl zu wählen sind;
und wobei schließlich M Wasserstoff, ein pharmazeutisch verträgliches Kation, Aroyl oder $C_1$- bis $C_6$-Alkoyl ist;

wobei das Verfahren die Umsetzung eines Carbamoylchlorides der Formel:

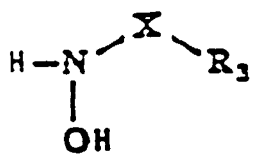

, wobei X und $R_3$ obiger Definition genügen, zu einem Hydroxyharnstoff der Formel:

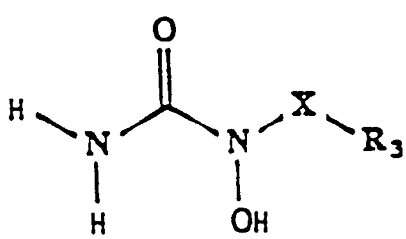

umfaßt, wobei X und $R_3$ obiger Definition entsprechen, welcher, sofern M nicht Wasserstoff ist, in ein Salz oder einen Ester der Formel:

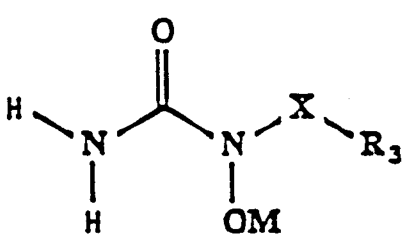

überführt wird, wobei M ein pharmazeutisch verträgliches Kation, Aroyl oder $C_1$- bis $C_6$-Alkoyl ist.

**10.** Das Verfahren gemäß Anspruch 9, wobei das Hydroxylamin durch Behandlung mit Natriumcyanat in den Hydroxyharnstoff überführt wird.

**11.** Das Verfahren gemäß Anspruch 9, wobei das Hydroxylamin in den Hydroxyharnstoff durch Behandlung mit Trimethylsilylisocyanat überführt wird.

**12.** Das Verfahren gemäß Anspruch 9, wobei das Hydroxylamin der Formel:

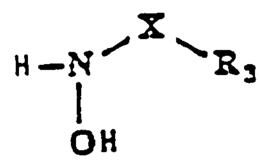

, wobei X und $R_3$ obiger Defintion entsprechen, mittels eines Verfahrens dargestellt wird, welches die Umwandlung eines Ketons oder Aldehyds der Formel $O=X\text{-}R_3$, wobei X und $R_3$ obiger Definition entsprechen, zu einem Oxim der Formel $HO\text{-}N=X\text{-}R_3$, wobei X und $R_3$ obiger Definition entsprechen, gefolgt von der Reduktion des Oxims zum Hydroxylamin der Formel:

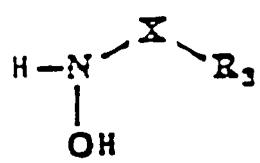

wobei X und $R_3$ obiger Definition entsprechen, umfaßt.

**13.** Das Verfahren gemäß Anspruch 12, wobei das Keton oder der Aldehyd durch Behandlung mit Hydroxylaminhydrochlorid in das Oxim überführt wird.

**14.** Das Verfahren gemäß Anspruch 12, wobei das Oxim mit einem Boran-Pyridin-Komplex zum Hydroxylamin reduziert wird.

**15.** Das Verfahren gemäß Anspruch 1 oder 9, wobei X $CH(CH_3)$ ist.

**16.** Das Verfahren gemäß Anspruch 1 oder 9, wobei X $CH_2$ ist.

**17.** Das Verfahren gemäß Anspruch 1 oder 9, wobei $Y_1$ Phenylmethoxy ist.

**18.** Das Verfahren gemäß Anspruch 1 oder 9, wobei $Y_1$ Butoxy ist.

**19.** Die Verwendung einer Verbindung der Formel:

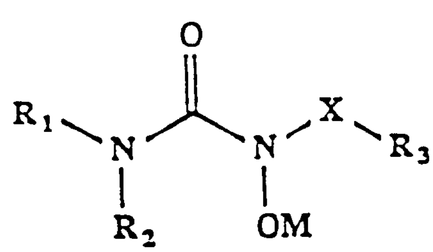

I

wobei $R_1$ und $R_2$ unabhängig voneinander aus Wasserstoff, $C_1$- bis $C_4$-Alkyl oder Hydroxyl gewählt sein können, aber $R_1$ und $R_2$ sind nicht beide gleichzeitig Hydroxyl;
wobei X aus $(C(R_4)_2)_m$ auswählbar ist, mit $m=1$ bis 3, und mit $R_4$, welcher bei jedem Auftreten unabhängig aus Wasserstoff oder $C_1$- bis $C_4$-Alkyl wählbar ist;
wobei $R_3$

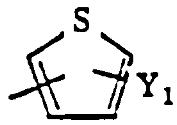

ist, mit $Y_1$ wählbar aus Wasserstoff, $C_1$- bis $C_6$-Alkyl, $C_2$- bis $C_6$- Alkenyl, $C_1$- bis $C_6$-Alkoxy, $C_3$- bis $C_8$-Cycloalkyl, Aryl, Aryloxy, Aroyl, $C_1$- bis $C_6$-Arylalkyl, $C_2$- bis $C_6$-Arylalkenyl, $C_1$- bis $C_6$–Arylalkoxy, $C_1$- bis $C_6$-Arylthioalkoxy, oder substituierte Derivate von Aryl, Aryloxy, Aroyl, $C_1$- bis $C_6$-Arylalkyl, $C_2$- bis $C_6$-Arylalkenyl, $C_1$- bis $C_6$-Arylalkoxy, $C_1$- bis $C_6$-Arylthioalkoxy, wobei die Substituenten aus Halogen, Nitro, Cyano, $C_1$- bis $C_6$-Alkyl, Alkoxy oder halogensubstituiertem Alkyl zu wählen sind;
und wobei schließlich M Wasserstoff, ein pharmazeutisch verträgliches Kation, Aroyl oder $C_1$- bis $C_6$-Alkoyl ist, zur Bereitung eines Medikamentes zur Inhibition von 5- oder 12-Lipoxygenase in einem Menschen oder niederen Tier im Bedarfsfall einer solchen Behandlung.

**20.** Die Verwendung einer Verbindung der Formel:

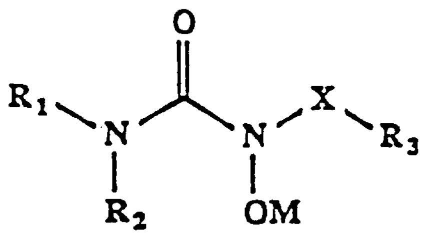

I

wobei $R_1$ und $R_2$ unabhängig voneinander aus Wasserstoff, $C_1$- bis $C_4$-Alkyl oder Hydroxyl gewählt sein können, aber $R_1$ und $R_2$ sind nicht beide gleichzeitig Hydroxyl;
wobei X aus $(C(R_4)_2)_m$ auswählbar ist, mit $m = 1$ bis 3, und mit $R_4$, welcher bei jedem Auftreten unabhängig aus Wasserstoff oder $C_1$- bis $C_4$-Alkyl wählbar ist;
wobei $R_3$

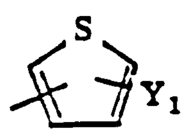

ist, mit $Y_1$ wählbar aus Wasserstoff, $C_1$- bis $C_6$-Alkyl, $C_2$- bis $C_6$-Alkenyl, $C_1$- bis $C_6$-Alkoxy, $C_3$- bis $C_8$-Cycloalkyl, Aryl, Aryloxy, Aroyl, $C_1$- bis $C_6$-Arylalkyl, $C_2$- bis $C_6$-Arylalkenyl, $C_1$- bis $C_6$–Arylalkoxy, $C_1$-bis $C_6$-Arylthioalkoxy, oder substituierte Derivate von Aryl, Aryloxy, Aroyl, $C_1$- bis $C_6$-Arylalkyl, $C_2$- bis $C_6$-Arylalkenyl, $C_1$- bis $C_6$-Arylalkoxy, $C_1$- bis $C_6$-Arylthioalkoxy, wobei die Substituenten aus Halogen, Nitro, Cyano, $C_1$- bis $C_6$-Alkyl, Alkoxy oder halogensubstituiertem Alkyl zu wählen sind;
und wobei schließlich M Wasserstoff, ein pharmazeutisch verträgliches Kation, Aroyl oder $C_1$- bis $C_6$-Alkoyl ist, zur Bereitung eines Medikamentes zur Behandlung von Asthma, allergischer Rhinitis, rheumatoider Arthritis, Gicht, dem Atemdisstressyndrom Erwachsener, dem Crohn'schen Leiden, Psoriase, endotoxinem Schock und/oder Ischämie-induziertem myocardischen Beschwerden bei einem Menschen oder einem niederem Tier im Bedarfsfall einer solchen Behandlung.

**Revendications**

**Revendications pour les Etats contractants suivants : BE, CH, DE, FR, GB, IT, LI, NL, SE**

1. Composé de formule :

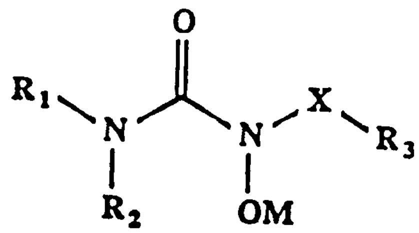

I

dans laquelle $R_1$ et $R_2$ sont indépendamment l'un de l'autre sélectionnés parmi l'hydrogène, un alkyle de $C_1$ à $C_4$, ou l'hydroxy, sous réserve que $R_1$ et $R_2$ ne soient pas simultanément un hydroxy;

dans laquelle X est sélectionné parmi les $(C(R_4)_2)_m$ avec m = 1 à 3;

où $R_4$ est sélectionné de façon indépendante à chacune de ses occurences parmi l'hydrogène ou un alkyle de $C_1$ à $C_4$,

dans laquelle $R_3$ est

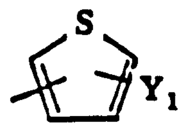

où $Y_1$ est sélectionné parmi l'hydrogène, un alkyle de $C_1$ à $C_6$, un alcényle de $C_2$ à $C_6$, un alcoxy de $C_1$ a $C_6$, un cycloalkyle de $C_3$ à $C_8$, un aryle, un aryloxy, un aroyle, un arylalkyle de $C_1$ à $C_6$, un arylalcényle de $C_2$ à $C_6$, un arylalcoxy de $C_1$ à $C_6$, un arylthioalcoxy de $C_1$ à $C_6$, ou des dérivés substitués d'aryle, d'aryloxy, d'aroyle, d'arylalkyle de $C_1$ à $C_6$, d'arylalcényle de $C_2$ à $C_6$, d'arylalcoxy de $C_1$ à $C_6$, d'arylthioalcoxy de $C_1$ à $C_6$, dans lesquels les substituants sont sélectionnés parmi halogéno, nitro, cyano, alkyle de $C_1$ à $C_6$, alcoxy ou alkyle halogénosubstitué;

et enfin dans laquelle M est un hydrogène, un cation pharmaceutiquement acceptable, un aroyle, ou un acyle de $C_1$ à $C_6$.

2. Composé selon la revendication 1 dans lequel $R_1$ et $R_2$ sont tous deux l'hydrogène.

3. Composé selon la revendication 1 dans lequel $R_1$ est l'hydrogène et $R_2$ est un hydroxy.

4. Composé selon la revendication 1 dans lequel X est $CH(CH_3)$.

5. Composé selon la revendication 1 dans lequel X est $CH_2$.

6. Composé selon la revendication 1 dans lequel $Y_1$ est phénylméthoxy.

7. Composé selon la revendication 1 dans lequel $Y_1$ est butoxy.

8. Composition pour l'inhibition de l'activité de la 5- ou 12-lipoxygénase chez un hôte humain ou animal inférieur ayant besoin d'un tel traitement, comprenant un composé selon la revendication 1 et un ou plusieurs supports, adjuvants ou véhicules pharmaceutiquement acceptables non toxiques.

9. Composition pour traiter l'asthme, la rhinite allergique, l'arthrite rhumatoïde, la goutte, le syndrome de détresse respiratoire de l'adulte, la maladie de Crohn, le psoriasis, le choc endotoxique, et / ou les troubles du myocarde induits par l'ischémie chez un humain ou un animal inférieur ayant besoin d'un tel traitement, comprenant un composé selon la revendication 1 et un ou plusieurs supports, adjuvants ou véhicules pharmaceutiquement acceptables non toxiques.

**10.** Utilisation d'un composé selon la revendication 1 pour préparer un médicament pour l'inhibition de l'activité de la 5- ou 12- lipoxygénase chez un hôte humain ou animal inférieur ayant besoin d'un tel traitement.

**11.** Utilisation d'un composé selon la revendication 1 pour préparer un médicament pour traiter l'asthme, la rhinite allergique, l'arthrite rhumatoïde, la goutte, le syndrome de détresse respiratoire de l'adulte, la maladie de Crohn, le psoriasis, le choc endotoxique, et / ou les troubles du myocarde induits par l'ischémie chez un humain ou un animal inférieur ayant besoin d'un tel traitement.

**Revendications pour les Etats contractants suivants : ES, GR**

**1.** Procédé pour préparer un composé de formule :

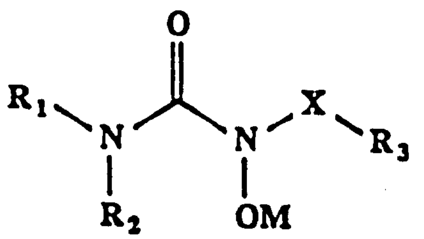

I

dans laquelle $R_1$ et $R_2$ sont indépendamment l'un de l'autre sélectionnés parmi l'hydrogène, un alkyle de $C_1$ à $C_4$, ou l'hydroxy, sous réserve que $R_1$ et $R_2$ ne soient pas simultanément un hydroxy;

dans laquelle X est sélectionné parmi les $(C(R_4)_2)_m$ avec m = 1 à 3;

où $R_4$ est sélectionné de façon indépendante à chacune de ses occurences parmi l'hydrogène ou un alkyle de $C_1$ à $C_4$,

dans laquelle $R_3$ est

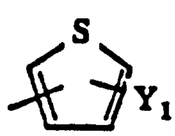

où $Y_1$ est sélectionné parmi l'hydrogène, un alkyle de $C_1$ à $C_6$, un alcényle de $C_2$ à $C_6$, un alcoxy de $C_1$ à $C_6$, un cycloalkyle de $C_3$ à $C_8$, un aryle, un aryloxy, un aroyle, un arylalkyle de $C_1$ à $C_6$, un arylalcényle de $C_2$ à $C_6$, un arylalcoxy de $C_1$ à $C_6$, un arylthioalcoxy de $C_1$ à $C_6$, ou des dérivés substitués d'aryle, d'aryloxy, d'aroyle, d'arylalkyle de $C_1$ à $C_6$, d'arylalcényle de $C_2$ à $C_6$, d'arylalcoxy de $C_1$ à $C_6$, d'arylthioalcoxy de $C_1$ à $C_6$, dans lesquels les substituants sont sélectionnés parmi halogéno, nitro, cyano, alkyle de $C_1$ à $C_6$, alcoxy ou alkyle halogénosubstitué;

et enfin dans laquelle M est un hydrogène, un cation pharmaceutiquement acceptable, un aroyle, ou un acyle de $C_1$ à $C_6$,

ledit procédé comprenant la réaction d'un chlorure de carbamoyle de formule :

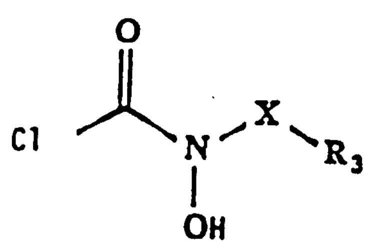

dans laquelle X et $R_3$ sont tels que définis ci-dessus, avec une amine de formule $HNR_1R_2$ dans laquelle $R_1$ et $R_2$ sont tels que définis ci-dessus pour donner un composé de formule :

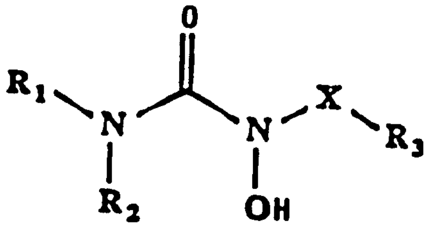

qui, si M n'est pas un hydrogène, est converti en un sel ou un ester de formule :

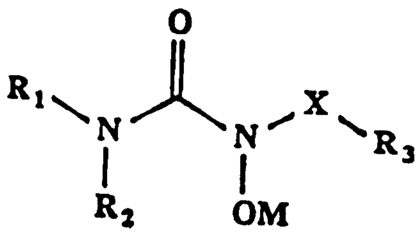

dans laquelle M est un cation pharmaceutiquement acceptable, un aroyle ou un acyle de $C_1$ à $C_6$.

**2.** Procédé de la revendication 1 dans lequel le chlorure de carbamoyle de formule :

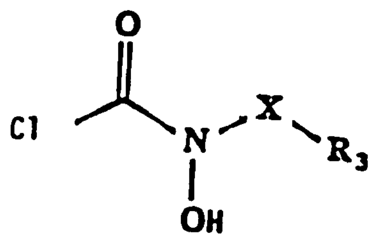

dans laquelle X et $R_3$ sont tels que définis ci-dessus est préparé par un procédé qui comprend la réaction d'une hydroxylamine de formule :

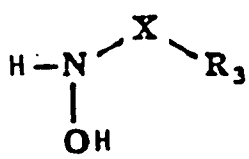

dans laquelle X et $R_3$ sont tels que définis ci-dessus, avec un agent formant un chlorure de carbamoyle pour donner un composé de formule :

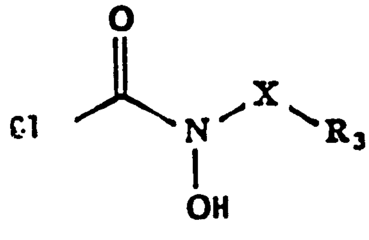

dans laquelle X, $R_1$, $R_2$ et $R_3$ sont tels que définis ci-dessus.

3. Procédé de la revendication 2 dans lequel l'hydroxylamine de formule :

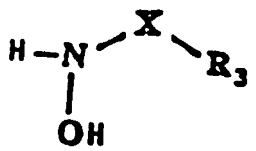

dans laquelle X et $R_3$ sont tels que définis ci-dessus est préparée par un procédé comprenant la conversion d'une cétone ou d'un aldéhyde de formule $O=X-R_3$, dans laquelle X et $R_3$ sont tels que définis ci-dessus en une oxime de formule $HO-N=X-R_3$ dans laquelle X et $R_3$ sont tels que définis ci-dessus, suivie de la réduction de l'oxime en hydroxylamine de formule

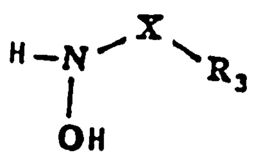

dans laquelle X et $R_3$ sont tels que définis ci-dessus.

4. Procédé de la revendication 2 dans lequel l'agent formant un chlorure de carbamoyle est le phosgène.

5. Procédé de la revendication 3 dans lequel la cétone ou l'aldéhyde est converti en oxime par traitement par le chlorhydrate d'hydroxylamine.

6. Procédé de la revendication 3 dans lequel l'oxime est réduite en hydroxylamine par le complexe borane - pyridine.

7. Procédé de la revendication 1 dans lequel $R_1$ et $R_2$ sont tous deux l'hydrogène.

8. Procédé de la revendication 1 dans lequel $R_1$ est l'hydrogène et $R_2$ est un hydroxy.

9. Procédé pour préparer un composé de formule :

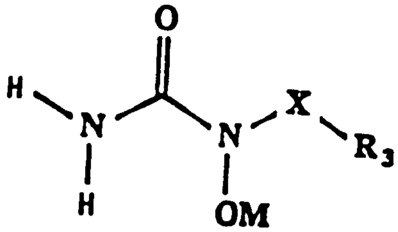

dans laquelle X est sélectionné parmi $(C(R_4)_2)_m$ avec m = 1 à 3;

où $R_4$ est sélectionné de façon indépendante à chacune de ses occurences parmi l'hydrogène ou un alkyle de $C_1$ à $C_4$,

dans laquelle $R_3$ est

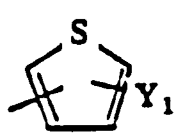

où $Y_1$ est sélectionné parmi l'hydrogène, un alkyle de $C_1$ à $C_6$, un alcényle de $C_2$ à $C_6$, un alcoxy

de $C_1$ à $C_6$, un cycloalkyle de $C_3$ à $C_8$, un aryle, un aryloxy, un aroyle, un arylalkyle de $C_1$ à $C_6$, un arylalcényle de $C_2$ à $C_6$, un arylalcoxy de $C_1$ à $C_6$, un arylthioalcoxy de $C_1$ à $C_6$, ou des dérivés substitués d'aryle, d'aryloxy, d'aroyle, d'arylalkyle de $C_1$ à $C_6$, d'arylalcényle de $C_2$ à $C_6$, d'arylalcoxy de $C_1$ à $C_6$, d'arylthioalcoxy de $C_1$ à $C_6$, dans lesquels les substituants sont sélectionnés parmi halogéno, nitro, cyano, alkyle de $C_1$ à $C_6$, alcoxy ou alkyle halogénosubstitué :

et enfin dans laquelle M est un hydrogène, un cation pharmaceutiquement acceptable un aroyle, ou un acyle de $C_1$ à $C_6$,

ledit procédé comprenant la réaction d'un chlorure de carbamoyle de formule :

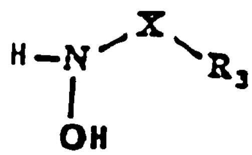

dans laquelle X et $R_3$ sont tels que définis ci-dessus, en hydroxyurée de formule :

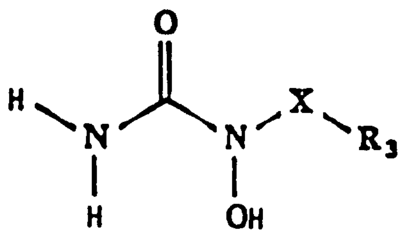

dans laquelle X et $R_3$ sont tels que définis ci-dessus, qui, quand M n'est pas un hydrogène est convertie en un sel ou un ester de formule :

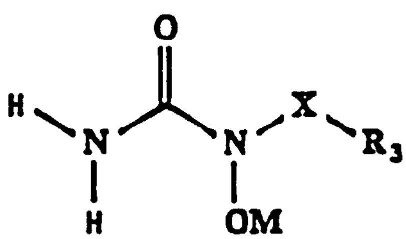

dans laquelle M est un cation pharmaceutiquement acceptable, un aroyle ou un acyle de $C_1$ à $C_6$.

**10.** Procédé de la revendication 9 dans lequel l'hydroxylamine est convertie en hydroxy urée par traitement avec le cyanate de sodium.

**11.** Procédé de la revendication 9 dans lequel l'hydroxylamine est convertie en hydroxy urée par traitement avec le triméthylsilylisocyanate.

**12.** Procédé de la revendication 9 dans lequel l'hydroxylamine de formule :

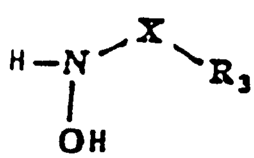

dans laquelle X et $R_3$ sont tels que définis ci-dessus est préparée par un procédé comprenant la conversion d'une cétone ou d'un aldéhyde de formule O=X-$R_3$ dans laquelle X et $R_3$ sont tels que

définis ci-dessus en une oxime de formule HO-N=X-$R_3$ dans laquelle X et $R_3$ sont tels que définis ci-dessus suivie de la réduction de l'oxime en hydroxylamine de formule

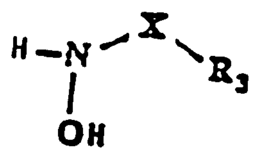

dans laquelle X et $R_3$ sont tels que définis ci-dessus.

**13.** Procédé de la revendication 12 dans lequel la cétone ou l'aldéhyde est converti en oxime par traitement par le chlorhydrate d'hydroxylamine.

**14.** Procédé de la revendication 12 dans lequel l'oxime est réduite en hydroxylamine par le complexe borane - pyridine.

**15.** Procédé de la revendication 1 ou 9 dans lequel X est $CH(CH_3)$.

**16.** Procédé de la revendication 1 ou 9 dans lequel X est $CH_2$.

**17.** Procédé de la revendication 1 ou 9 dans lequel $Y_1$ est phénylméthoxy.

**18.** Procédé de la revendication 1 ou 9 dans lequel $Y_1$ est butoxy.

**19.** Utilisation d'un composé de formule :

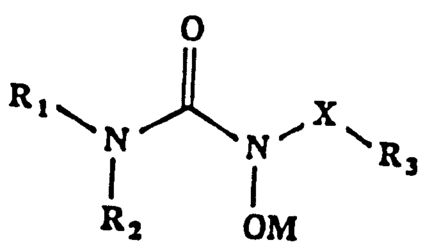

I

dans laquelle $R_1$ et $R_2$ sont indépendamment l'un de l'autre sélectionnés parmi l'hydrogène, un alkyle de $C_1$ a $C_4$, ou l'hydroxy, sous réserve que $R_1$ et $R_2$ ne soient pas simultanément un hydroxy :

dans laquelle X est sélectionné parmi les $(C(R_4)_2)_m$ avec m = 1 à 3 :

où $R_4$ est sélectionné de façon indépendante à chacune de ses occurences parmi l'hydrogène ou un alkyle de $C_1$ à $C_4$.

dans laquelle $R_3$ est

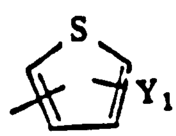

où $Y_1$ est sélectionné parmi l'hydrogène, un alkyle de $C_1$ à $C_6$, un alcényle de $C_2$ à $C_6$, un alcoxy de $C_1$ à $C_6$, un cycloalkyle de $C_3$ à $C_8$, un aryle, un aryloxy, un aroyle, un arylalkyle de $C_1$ à $C_6$, un arylalcényle de $C_2$ à $C_6$, un arylalcoxy de $C_1$ à $C_6$ un arylthioalcoxy de $C_1$ à $C_6$, ou des dérivés substitués d'aryle, d'aryloxy, d'aroyle, d'arylalkyle de $C_1$ à $C_6$, d'arylalcényle de $C_2$ à $C_6$ d'arylalcoxy de $C_1$ à $C_6$, d'arylthioalcoxy de $C_1$ à $C_6$, dans lesquels les substituants sont sélectionnés parmi halogéno, nitro, cyano, alkyle de $C_1$ à $C_6$, alcoxy ou alkyle halogénosubstitué ;

et enfin dans laquelle M est un hydrogène, un cation pharmaceutiquement acceptable, un aroyle,

ou un acyle de $C_1$ à $C_6$ pour la préparation d'un médicament pour l'inhibition de la 5- ou 12-lipoxygénase chez un humain ou un animal inférieur ayant besoin d'un tel traitement.

**20.** Utilisation d'un composé de formule :

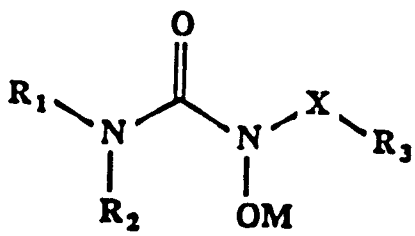

I

dans laquelle $R_1$ et $R_2$ sont indépendamment l'un de l'autre sélectionnés parmi l'hydrogène, un alkyle de $C_1$ à $C_4$, ou l'hydroxy, sous réserve que $R_1$ et $R_2$ ne soient pas simultanément un hydroxy :

dans laquelle X est sélectionné parmi les $(C(R_4)_2)_m$ avec m = 1 à 3 :

où $R_4$ est sélectionné de façon indépendante à chacune de ses occurences parmi l'hydrogène ou un alkyle de $C_1$ à $C_4$,

dans laquelle $R_3$ est

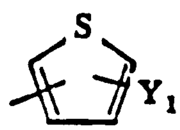

où $Y_1$ est sélectionné parmi l'hydrogène un alkyle de $C_1$ à $C_6$, un alcényle de $C_2$ à $C_6$, un alcoxy de $C_1$ à $C_6$, un cycloalkyle de $C_3$ à $C_8$, un aryle, un aryloxy, un aroyle, un arylalkyle de $C_1$ à $C_6$, un arylalcényle de $C_2$ à $C_6$, un arylalcoxy de $C_1$ à $C_6$, un arylthioalcoxy de $C_1$ à $C_6$, ou des dérivés substitués d'aryle, d'aryloxy, d'aroyle, d'arylalkyle de $C_1$ à $C_6$, d'arylalcényle de $C_2$ à $C_6$, d'arylalcoxy de $C_1$ à $C_6$, d'arylthioalcoxy de $C_1$ à $C_6$, dans lesquels les substituants sont sélectionnés parmi halogéno, nitro, cyano, alkyle de $C_1$ à $C_6$, alcoxy ou alkyle halogénosubstitué :

et enfin dans laquelle M est un hydrogène, un cation pharmaceutiquement acceptable un aroyle, ou un acyle de $C_1$ à $C_6$, pour la préparation d'un médicament pour traiter l'asthme, la rhinite allergique, l'arthrite rhumatoïde, la goutte, le syndrome de détresse respiratoire de l'adulte, la maladie de Crohn, le psoriasis, le choc endotoxique, et / ou les troubles du myocarde induits par l'ischémie chez un humain ou un animal inférieur ayant besoin d'un tel traitement.